(19) Europäisches Patentamt
European Patent Office
Office européen des brevets

(11) Veröffentlichungsnummer: **0 316 783 B1**

(12) **EUROPÄISCHE PATENTSCHRIFT**

(45) Veröffentlichungstag der Patentschrift: **18.03.92**

(51) Int. Cl.5: **C07D 261/08**, C07D 213/48, C07D 213/50, C07D 239/26, //C07C45/30

(21) Anmeldenummer: **88118788.4**

(22) Anmeldetag: **11.11.88**

# (54) Verfahren zur Herstellung von heterocyclisch-aromatischen Aldehyden und Ketonen.

(30) Priorität: **17.11.87 DE 3738909**

(43) Veröffentlichungstag der Anmeldung: **24.05.89 Patentblatt 89/21**

(45) Bekanntmachung des Hinweises auf die Patenterteilung: **18.03.92 Patentblatt 92/12**

(84) Benannte Vertragsstaaten: **BE CH DE FR GB IT LI NL**

(56) Entgegenhaltungen:
**GB-A- 2 017 091**

**THE JOURNAL OF ORGANIC CHEMISTRY, Band 52, Nr. 12, 12. Juni 1987, Seiten 2559-2562, American Chemical Society, Washington DC, US; P.L. ANELLI et al.: "Fast and selective oxidation of primary alcohols to aldehydes or to carboxylic acids and of secondary alcohols to ketones mediated by oxoammonium salts under two-phase conditions"**

**JOURNAL OF ORGANIC CHEMISTRY, Band 50, Nr. 8, April 1985, Seiten 1332-1334, American Chemical Society, Washington DC, US; T. MIYAZAWA et al.: "Selective oxidation of alcohols by oxoaminium salts (R2N=O+X-)**

(73) Patentinhaber: **BASF Aktiengesellschaft**
**Carl-Bosch-Strasse 38**
**W-6700 Ludwigshafen(DE)**

(72) Erfinder: **Kuekenhoehner, Thomas, Dr.**
**Seidelstrasse 2**
**W-6710 Frankenthal(DE)**
Erfinder: **Goetz, Norbert, Dr.**
**Schoefferstrasse 25**
**W-6250 Worms 1(DE)**
Erfinder: **Theobald, Hans, Dr.**
**Oueichstrasse 6**
**W-6703 Limburgerhof(DE)**
Erfinder: **Knaus, Guenter H., Dr.**
**Wanderstrasse 37**
**W-6700 Ludwigshafen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft ein neues Verfahren zur Herstellung von heterocyclisch-aromatischen (heteroaromatischen) Aldehyden und Ketonen der allgemeinen Formel I

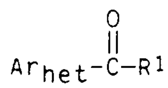

I

in der $Ar_{het}$ einen 5- oder 6-gliedrigen heteroaromatischen Rest mit mindestens einem Stickstoffatom und $R^1$ Wasserstoff oder einen $C_1$-$C_{20}$-Alkylrest bedeutet, durch Oxidation der entsprechenden 1-Hydroxyalkylverbindungen II

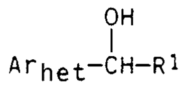

II

Es ist allgemein bekannt, Aldehyde und Ketone durch Oxidation der entsprechenden primären oder sekundären Alkohole herzustellen

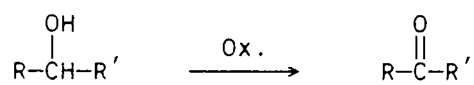


Während jedoch die Oxidation aliphatischer Alkohole meistens relativ problemlos ist und während zahlreiche isoaromatische Alkohole wie Benzylalkohol bereits durch die Einwirkung von Luftsauerstoff in die Aldehyde überführt werden, bereitet die Herstellung heteroaromatischer Aldehyde aus den entsprechenden Hydroxymethylverbindungen Schwierigkeiten. So verwendet man beispielsweise im Falle der Pyridinaldehyde Selendioxid, Bleitetraacetat oder Mangandioxid als Oxidationsmittel (vgl. Ullmanns Encyklopädie der Technischen Chemie, 3. Auflage, Band 14, 1963, Seite 478). Ähnliches gilt für die Heteroarylalkylketone. So lassen sich z.B. die besonders wichtigen Pyridylalkylketone durch Oxidation der entsprechenden Alkohole mit $CrO_3/H_2SO_4$ oder $MnO_2$ herstellen (V. Deljac et al., Acta Pharm. Jugosl. 32 (4), 267-74 (1982), Chem.Abstr. 98, 174241t und US-A 4 098 908). Jedoch sind die hierbei erzielbaren Ausbeuten und Selektivitäten meistens unbefriedigend, ganz abgesehen davon, daß die Verwendung dieser teuren und physiologisch nicht unbedenklichen, in fester Form einzusetzenden Oxidationsmittel erhebliche verfahrenstechnische Probleme aufwirft.

Weiterhin ist es aus der Arbeit von Anelli et al (J.Org.Chem., Band 52, 1987, Seiten 2559-2562) bekannt, für die Oxidation von einigen aliphatischen und isoaromatischen Alkoholen zu den entsprechenden Carbonylverbindungen Natriumhypochlorit (NaOCl) in Verbindung mit katalytischen Mengen der Piperidinderivate

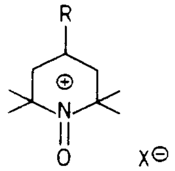

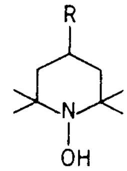

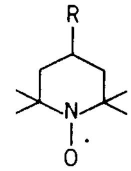

$R = H; CH_3-O-$

$X = Cl; Br$

und von Kaliumbromid erfolgreich zu verwenden, jedoch finden heteroaromatische Hydroxymethylverbindungen bzw. Aldehyde und Ketone in diesen Untersuchungen keine Erwähnung.

Der Erfindung lag die Aufgabe zugrunde, bestimmte heteroaromatische Aldehyde und Ketone auf einfachere und wirtschaftlichere Weise zugänglich zu machen als bisher.

Demgemäß wurde ein Verfahren zur Herstellung von heterocyclisch-aromatischen (heteroaromatischen) Aldehyden und Ketonen der allgemeinen Formel I

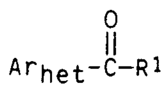

I

in der $Ar_{het}$ einen 5- oder 6-gliedrigen heteroaromatischen Rest mit mindestens einem Stickstoffatom und $R^1$ Wasserstoff oder einen $C_1$-$C_{20}$-Alkylrest bedeutet, durch Oxidation der entsprechenden 1-Hydroxymethylverbindungen II

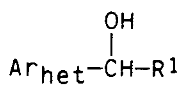

II

gefunden, welches dadurch gekennzeichnet ist, daß man hierzu als Oxidationsmittel ein anorganisches oder organisches Hypochlorit oder Hypobromit (III) in Verbindung mit einem $\alpha,\alpha,\omega,\omega$-Tetraalkylcycloalkan der allgemeinen Formel IV

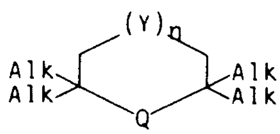

IV

verwendet, in der Alk gleiche oder verschiedene $C_1$-$C_4$-Alkylgruppen bezeichnet, Q für eine der Gruppierungen

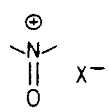 ; 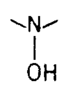 oder 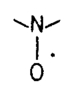

steht, wobei $X^-$ ein Anion bedeutet, n 0 oder 1 ist und Y für Sauerstoff, die Carbonylgruppe oder einen Rest

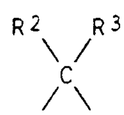

steht, in welchem $R^2$ und $R^3$ Wasserstoff, die Hydroxylgruppe oder C-organische oder O-organische Reste bedeuten, die auch miteinander zu einem fünf- oder sechsgliedrigen Ring verbunden sein können.

Ferner wurde gefunden, daß diese Reaktion besonders gut gelingt, wenn man sie zusätzlich in Gegenwart katalytischer Mengen eines anorganischen Bromides vornimmt, wenn man sie unabhängig hiervon in einem zweiphasigen wäßrig-organischen Lösungsmittelsystem ausführt und wenn man einen pH-Wert von 5 bis 10 einhält.

Der Rest $Ar_{het}$ in den Verfahrensprodukten I bzw. den Ausgangsverbindungen II entspricht folgenden heteroaromatischen Stammkörpern:

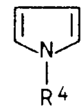

Pyrrol

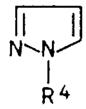

Pyrazol

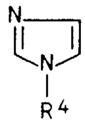

Imidazol

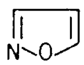

Isoxazol

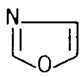

Oxazol

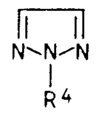 1,2,3-Triazol

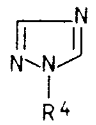 1,2,4-Triazol

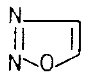 1,2,3-Oxadiazol

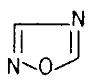 1,2,4-Oxadiazol

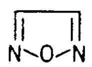 1,2,5-Oxadiazol

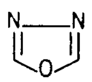 1,3,4-Oxadiazol

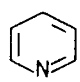 Pyridin

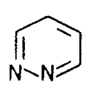 Pyridazin

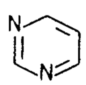 Pyrimidin

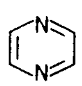 Pyrazin

$R^4$: $C_1$-$C_4$-Alkyl; Benzyl

In den entsprechenden Ausgangsverbindungen II kann die 1-Hydroxyalkylgruppe an ein beliebiges C-Atom des Ringes gebunden sein und die übrigen C-Atome können beliebige inerte, also z.B. nicht selber oxidierbare organische, vorzugsweise über ein C-Atom gebundene Reste tragen, beispielsweise

- $C_1$-$C_8$-Alkylgruppen wie die Methyl, Ethyl- und Isopropylgruppe;
- Cycloalkylgruppen mit 3 bis 12 Ringgliedern, wie die Cyclopentyl- und Cyclohexylgruppe;
- Oxacycloalkylgruppen mit 5 und 6 Ringgliedern wie die 2-, 3- und 4-Oxacyclohexylgruppen und die 2- und 3-Oxacyclopentylgruppe;
- Cycloalkylalkylgruppen wie die 1- und 2-Cyclohexylethylgruppe;
- Arylgruppen wie die Phenyl-, $\alpha$-Naphthyl- und $\beta$-Naphthylgruppe;
- Aralkylgruppen wie die Benzyl- und die 1- oder 2-Phenylethylgruppe;
- Acylgruppen $R'$-CO- wobei $R'$ eine der vorgenannten Gruppen bedeutet, beispielsweise die Acetyl- und die Benzoylgruppe;
- Organyloxygruppen $R'$-O-, wobei $R'$ eine der vorgenannten Alkyl-, Cycloalkyl-, Oxacycloalkyl; Cycloalkylalkyl-, Aryl- oder Aralkylgruppen bedeutet;
- Oxycarbonylgruppen $R'$-O-CO- wie die Methoxy- und Phenoxycarbonylgruppe und
- Carbonyloxygruppen $R'$-CO-O-, wobei $R'$ einen der vorstehend definierten Reste bedeutet.

Ferner können Oxazol, Isoxazol, 1,2,3-, 1,2,5-Oxadiazol und die Stammkörper der Pyridinreihe auch anellierte Ringe tragen wie beispielsweise im Benzoxazol, Benzisoxazol und im Chinolin, und außerdem können die vorgenannten Gruppen ihrerseits durch Fluor, Chlor, Brom, die Cyangruppe und $C_1$-$C_4$-Alkoxygruppen substituiert sein.

$R^1$ kann Wasserstoff oder einen $C_1$-$C_{20}$-Alkylrest bedeuten.

Die Ausgangsverbindungen II sind bekannt oder nach bekannten Methoden erhältlich.

Die im Hinblick auf die Verfahrensprodukte besonders wichtigen Hydroxymethylisoxazole IIa-IIc lassen sich beispielsweise wie folgt herstellen:

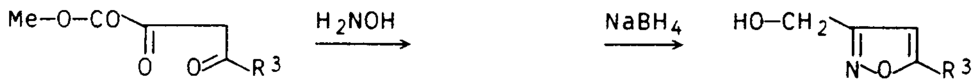


IIa

(J.Org.Chem. 26 (1961), S. 1514)

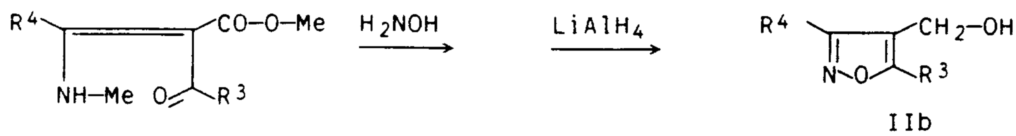


IIb

(Ber. 42 (1903), S. 3912)

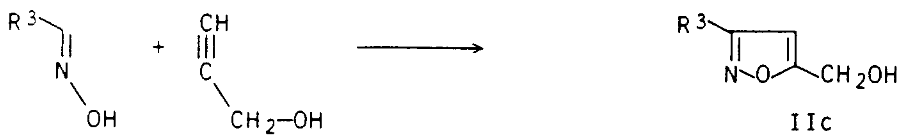

IIc

(DE-A 27 54 832)

$R^3$, $R^4$ = organische Reste

Ähnliches gilt für die übrigen Ausgangsverbindungen II.

Als wichtige Verbindungen II, welche Vorstufen der Zwischenprodukte I für die Synthese von bekannten Pflanzenschutzmitteln (s. z.B. DE-A 36 09 181) dienen, seien genannt:

5-Hydroxymethyl-3-methyl-isoxazol
5-Hydroxymethyl-3-ethyl-isoxazol
5-Hydroxymethyl-3-propyl-isoxazol
5-Hydroxymethyl-3-isopropyl-isoxazol
5-Hydroxymethyl-3-n-butyl-isoxazol
5-Hydroxymethyl-3-isobutyl-isoxazol
5-Hydroxymethyl-3-sec.-butyl-isoxazol
5-Hydroxymethyl-3-tert.-butyl-isoxazol
5-Hydroxymethyl-3-ethoxycarbonyl-isoxazol
5-Hydroxymethyl-3-cyclopropyl-isoxazol
5-Hydroxymethyl-3-cyclobutyl-isoxazol
5-Hydroxymethyl-3-cyclopentyl-isoxazol
5-Hydroxymethyl-3-cyclohexyl-isoxazol
5-Hydroxymethyl-3-(2-oxacyclohexyl)-isoxazol
5-Hydroxymethyl-3-(3-oxacyclohexyl)-isoxazol
5-Hydroxymethyl-3-(4-oxacyclopentyl)-isoxazol
5-Hydroxymethyl-3-(2-oxacyclopentyl)-isoxazol
5-Hydroxymethyl-3-(3-oxacyclopentyl)-isoxazol
4-Hydroxymethyl-3,5-dimethyl-isoxazol
3-Hydroxymethyl-5-isopropyl-isoxazol
5-(1-Hydroxypentyl)-3-methyl-isoxazol

5-(1-Hydroxyhexyl)-3-ethoxycarbonyl-isoxazol
1-Benzyl-2-(1-hydroxyhexyl)-imidazol
3-Hydroxymethyl-pyridin
2-Hydroxymethyl-pyridin
4-Hydroxymethyl-pyridin
3-(1-Hydroxyhexyl)-pyridin
3-(2-Ethyl-1-hydroxypentyl)-pyridin
3-(1-Hydroxypentyl)-pyridin

Als Oxidationsmittel dienen die anorganischen oder organischen Hypochlorite und -bromite (III, im folgenden als Hypohalogenite bezeichnet).

Als anorganische Verbindungen dieser Art kommen besonders Natriumhypochlorit sowie daneben Kaliumhypochlorit, Calciumhypochlorit und Natriumhypobromit in Betracht. Man setzt sie vorzugsweise in Form von etwa 5 bis 20 gew.%igen wäßrigen Lösungen ein, die man aus den reinen Salzen oder aus den (Erd-)Alkalihydroxidlösungen und Chlor bzw. Brom bereiten kann.

Als organische Hypochlorite und Hypobromite eignen sich in erster Linie tertiäre Hypohalogenite, darunter vor allem tert.-Butylhypochlorit.

Für die vollständige Oxidation von II zu I sind mindestens äquimolare Mengen von III, bezogen auf die Menge von II, erforderlich, jedoch empfiehlt es sich im allgemeinen, III im bis zu 50 mol%igen Überschuß über II einzusetzen. Handelt es sich um sehr oxidationsempfindliche Verbindungen II oder I, kann es auch ratsam sein, den Umsatz durch Verwendung unterstöchiometrischer Mengen an III - etwa 50 bis 90 % III pro Mol II - zu begrenzen.

Wesentlich für das erfindungsgemäße Oxidationsverfahren ist die Mitverwendung der Verbindungen IV, die als katalytische Sauerstoffüberträger fungieren. Im einzelnen handelt es sich hierbei in erster Linie um die Piperidinderivate IVa-IVc

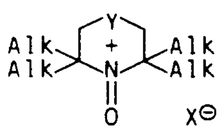
IVa

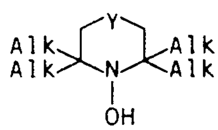
IVb

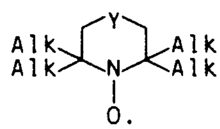
IVc

wobei die 2,2,6,6-Tetramethylverbindungen die größte Bedeutung haben, weil sie am leichtesten zugänglich sind. Die Art der Gruppierung Y hat nur einen untergeordneten Einfluß auf die Reaktion, so daß sie im Prinzip beliebig sein kann, jedoch bevorzugt man aus wirtschaftlichen Gründen einfache Verbindungen IV, in denen Y Sauerstoff, die Oxogruppe, die Methylengruppe, die 1,1-Ethylengruppe, die Hydroxymethylengruppe oder die Methoxymethylengruppe bedeutet.

Das Anion $X^{\ominus}$ kann grundsätzlich beliebig sein; aus praktischen Gründen werden aber Sulfat, p-Tolylsulfonat, Acetat und vor allem Chlorid und Bromid bevorzugt.

Die Verbindungen IVa bis IVc, die in bekannter Weise erhältlich sind (vgl. z.B. Synthesis, 1971, S. 191), sind stabil und können als solche eingesetzt werden. Zusammen mit den Hypohalogeniten III und den zu oxidierenden Alkoholen II gehen sie jedoch im Verlaufe des katalytischen Oxidationszyklus ineinander über, wobei IVa vermutlich das unmittelbar oxidierende Agens ist.

Man kann IVa-IVc direkt einsetzen oder in situ aus den entsprechenden Piperidinen und einem Oxidationsmittel entstehen lassen. Als Oxidationsmittel eignen sich vor allem peroxidische Mittel wie Wasserstoffperoxid, zweckmäßigerweise zusammen mit Natriumwolframat oder ähnlichen metalloxidischen Verbindungen hoher Oxidationsstufe, und besonders organische Peroxide wie m-Chlorperbenzoesäure (MCPBA).

Alle für die Piperidinderivate IVa-IVc gegebenen Erläuterungen gelten sinngemäß auch für die Pyrrolidinverbindungen IVd-IVf, so daß sich gesonderte Ausführungen hierüber erübrigen.

Die zu verwendende Menge der Verbindungen IV ist prinzipiell beliebig. Da sie aber katalytisch wirken, setzt man sie vorzugsweise in Mengen von 0,1 bis 20 mol% pro Mol II ein. Bei Mengen unter 0,1 mol% verlangsamt sich die Reaktion zu sehr, und bei Mengen über 20 mol% kann man im allgemeinen nicht mehr mit nennenswerten Vorteilen rechnen.

Da bei der Reaktion Wasser gebildet wird und da man die Hypohalogenite III vorzugsweise in wäßriger Lösung einsetzt, liegt stets eine wäßrige Phase vor. Es empfiehlt sich aber, durch Mitverwendung wasserunlöslicher Lösungsmittel wie Methylenchlorid, Chloroform, Ethylacetat, Butylacetat, Diethylether

oder Toluol auch für eine organische Phase zu sorgen, in welcher sich die organophilen Reaktionspartner lösen und welche insbesondere die Aldehyde I aufzunehmen und in gewissem Ausmaß vor Folgereaktionen zu schützen vermag. Die Menge dieser wasserunlöslichen Lösungsmittel ist nicht kritisch, liegt aber im allgemeinen zwischen 1 und 10 kg pro kg II.

Weiterhin ist es vorteilhaft, den pH-Wert der wäßrigen Phase auf einen Wert zwischen 5 und 10 einzustellen und durch Mitverwendung eines Pufferungsmittels wie Natriumhydrogencarbonat, Natriumacetat oder Dinatriumhydrogenphosphat möglichst konstant zu halten, denn im stärker sauren Bereich sind die Hypohalogenite III weniger stabil und in stärker alkalischem Bereich können die gebildeten Aldehyde I unerwünschte Folgereaktionen eingehen.

Ferner wird die erfindungsgemäße Reaktion durch Bromidionen begünstigt welche man zweckmäßigerweise in Form von Natrium- oder Kaliumbromid einsetzt und in Mengen von 1 bis 10 mol% pro Mol II verwendet. Dienen Hypobromite als Oxidationsmittel, bilden sich die Bromidionen von selbst, so daß es keines gesonderten Zusatzes bedarf.

Für die Reaktionstemperaturen empfiehlt sich der Bereich von 0 bis 100 °C, vorzugsweise von 0 bis 40 °C. Im allgemeinen wird man unter Atmosphärendruck oder, falls tiefsiedende Lösungsmittel verwendet werden, unter dem Eigendruck des Reaktionsgemisches arbeiten.

In verfahrenstechnischer Hinsicht ist es zweckmäßig, alle Komponenten mit Ausnahme von III in einem wäßrig-organischen zweiphasigen System vorzulegen und dann allmählich, etwa im Lauf von 0,5 bis 10 Stunden, mit III zu versetzen. Im übrigen weist das Verfahren, welches nach den üblichen Techniken auch kontinuierlich gestaltet werden kann, keine verfahrenstechnischen Besonderheiten auf, so daß nähere Angaben hierzu entbehrlich sind. Das gleiche gilt für die Aufarbeitung der Reaktionsgemische, bei der man die Verfahrensprodukte I wie üblich aus der organischen Phase isoliert. Die zurückbleibende wäßrige Phase, in der sich überschüssiges Oxidationsmittel, Katalysatoren und gegebenenfalls Puffersubstanzen befinden, kann für weitere Reaktionsansätze verwendet werden. Sofern die organische Phase noch Hypohalogenite enthält, können diese mit Reduktionsmitteln wie Eisen-II-sulfat zerstört werden.

Man erhält die heteroaromatischen Aldehyde und Ketone I, die u.a. wertvolle Zwischenprodukte für die Herstellung von Pflanzenschutzmitteln sind, in unerwartet hohen Ausbeuten von etwa bis zu 85 %.

Beispiel 1

Herstellung von 5-Formyl-3-tert.-butyl-isoxazol (I/1)

Eine Mischung aus 465 g (3 mol) 5-Hydroxymethyl-3-tert.-butyl-isoxazol, 9,4 g (0,06 mol) 2,2,6,6-Tetramethylpiperidin-1-oxyl, 17,9 g (0,15 mol) Kaliumbromid, 46,8 g (0,3 mol) $NaH_2PO_4 \cdot 2\,H_2O$, 53,4 g (0,3 mol) $Na_2HPO_4 \cdot 2\,H_2O$, 1800 ml Methylenchlorid und 1800 ml Wasser wurde bei 20 °C unter intensivem Rühren im Laufe von 3,5 h mit 1756 g einer 14 gew.%igen wäßrigen Natriumhypochloritlösung (3,3 mol NaOCl) versetzt. Der pH-Wert des Reaktionsgemisches stellte sich während der gesamten Reaktionsdauer ziemlich konstant auf 6,5 bis 7,5 ein.

Anschließend wurde die organische Phase abgetrennt und die wäßrige Phase mit Methylenchlorid extrahiert. Die vereinigten organischen Phasen wurden mit wäßriger $NaHCO_3$-Lösung gewaschen und wie üblich auf das Verfahrensprodukt I/1 aufgearbeitet.
Ausbeute: 77 %
Sdp.: 82-84 °C/0,01 mbar
Smp.: 42-43 °C

Beispiel 2

Herstellung von 5-Formyl-3-isopropyl-isoxazol (I/2)

Eine Mischung aus 14,1 g (0,1 mol) 5-Hydroxymethyl-3-isopropyl-isoxazol, 1,6 g (0,01 mol) 2,2,6,6-Tetramethylpiperidin-1-oxyl, 16,5 g (0,1 mol) $NaH_2PO_4 \cdot 2\,H_2O$, 60 ml Methylenchlorid und 60 ml Wasser wurde bei 20 °C im Laufe von 75 min unter Rühren mit 10,9 g (0,1 mol) tert.-Butylhypochlorit versetzt und noch 30 min nachgerührt. Der pH-Wert betrug etwa 8.

Die wäßrige Phase wurde mit Methylenchlorid extrahiert und die Extraktphase wurde mit der ursprünglichen organischen Phase vereinigt, mit 30 ml verdünnter Salzsäure, danach mit 30 ml gesättigter wäßriger Eisensulfatsulfatlösung und schließlich mit $NaHCO_3$-Lösung gewaschen und wie üblich auf I/2 aufgearbeitet.
Ausbeute: 73 %
Sdp.: 74 °C/4 mbar

Beispiel 3

Herstellung von 5-Formyl-3-isopropyl-isoxazol (I/2)

Zu einer Lösung aus 0,3 g (2,3 mmol) 2,2,5,5-Tetramethylpyrrolidin und 10 ml Methylenchlorid wurde im Laufe von 15 min bei 20 °C eine Lösung von 1,6 g (9,2 mmol) m-Chlorperbenzoesäure und 25 ml Methylenchlorid getropft, wonach noch 30 min nachgerührt wurde. Zu der so erhaltenen Katalysatorlösung wurden 40 ml Methylenchlorid, 70 ml Wasser, 0,69 g (5,8 mmol) Kaliumbromid, 16,2 g (0,115 Mol) 5-Hydroxymethyl-3-isopropyl-isoxazol, 1,8 g (11,5 mmol) $NaH_2PO_4 \cdot 2H_2O$ und 2,0 g (11,5 mmol) $Na_2HPO_4$ hinzugefügt, wonach zu diesem Gemisch im Laufe von 2 Stunden bei 25 °C unter intensivem Rühren 67,3 g (0,126 Mol) der Natriumhypochloritlösung gemäß Beispiel 1 gegeben wurde. Es wurde 15 min nachgerührt, die organische Phase wurde abgetrennt und die wäßrige Phase mit Methylenchlorid extrahiert. Die vereinigten organischen Phasen wurden mit wäßriger $NaHCO_3$-Lösung gewaschen und wie üblich auf das Verfahrensprodukt I/2 aufgearbeitet.
Ausbeute: 64 %.

Beispiele 4 bis 24

Herstellung verschiedener Verbindungen I

Allgemeine Arbeitsbedingungen (Beispiel 4)

Zur Bereitung eines katalytisch aktiven Piperidinderivates IV wurde eine Lösung aus 9,3 (0,06 mol) 2,2,6,6-Tetramethylpiperidin-4-on und 150 ml Methylenchlorid im Laufe von 15 min bei 20 °C mit einer Lösung aus 25,9 g (0,15 mol) m-Chlorperbenzoesäure und 300 ml Methylenchlorid versetzt und noch 30 min nachgerührt. Zu der so erhaltenen Katalysatorlösung wurden 423 g (3 mol) 5-Hydroxymethyl-3-isopropyl-isoxazol, 18 g (0,15 mol) Kaliumbromid, 46,8 g (0,3 mol) $NaH_2PO_4 \cdot 2\,H_2O$, 53,4 g (0,3 mol) $Na_2HPO_4 \cdot 2\,H_2O$, 1350 ml Methylenchlorid und 1800 ml Wasser hinzugefügt, wonach zu diesem Gemisch im Laufe von 4 h bei 25 °C unter intensivem Rühren 1756 g (3,3 mol NaOCl) der Natriumhypochloritlösung gemäß Beispiel 1 gegeben wurden.

Auf analoge Weise wurden die Beispiele 5 bis 24 ausgeführt, wobei die Aufarbeitung jeweils wie in Beispiel 1 erfolgte und wobei sich in allen Fällen ein pH-Wert von 6,5-8,0 einstellte.

Die Einzelheiten zu diesen Versuchen sind der nachstehenden Tabelle zu entnehmen.

| Bsp. | Edukt II | Produkt I Verb. | Ausbeute [%] | Sdp. [°C/mbar] | Abweichungen gegen Beispiel 4 |
|---|---|---|---|---|---|
| 4 | 5-Hydroxymethyl-3-isopropyl-isoxazol | I/ 2 | 82 | s.Bsp.2 | - |
| 5 | 5-Hydroxymethyl-3-isopropyl-isoxazol | I/ 2 | 80 | s.Bsp.2 | -piperidin statt -piperidin-4-on (IV) |
| 6 | 5-Hydroxymethyl-3-isopropyl-isoxazol | I/ 2 | 77 | s.Bsp.2 | ohne KBr |
| 7 | 5-Hydroxymethyl-3-isopropyl-isoxazol | I/ 2 | 72 | s.Bsp.2 | wie Bsp.5; bei 0°C |
| 8 | 5-Hydroxymethyl-3-isopropyl-isoxazol | I/ 2 | 75 | s.Bsp.2 | bei 0°C |
| 9 | 5-Hydroxymethyl-3-isopropyl-isoxazol | I/ 2 | 71 | s.Bsp.2 | wie Bsp.5; Ethylacetat statt $CH_2Cl_2$ |
| 10 | 5-Hydroxymethyl-3-isopropyl-isoxazol | I/ 2 | 69 | s.Bsp.2 | wie Bsp.5; Toluol statt $CH_2Cl_2$ |
| 11 | 5-Hydroxymethyl-3-ethyl-isoxazol | I/ 3 | 81 | 52-53/1,0 | |
| 12 | 5-Hydroxymethyl-3-methyl-isoxazol | I/ 4 | 62 | 56/3,5 | |
| 13 | 5-Hydroxymethyl-3-isobutyl-isoxazol | I/ 5 | 77 | 84-88/0,3 | |
| 14 | 5-Hydroxymethyl-3-ethoxycarbonyl-isoxazol | I/ 6 | 46 | 95-102/0,3-0,4 | |
| 15 | 5-Hydroxymethyl-3-(2-oxacyclohexyl)-isoxazol | I/ 7 | 76 | 95/0,01 | |
| 16 | 5-Hydroxymethyl-3-tert.-butyl-isoxazol | I/ 8 | 84 | s. Bsp. 1 | |
| 17 | 5-Hydroxymethyl-3-(3-oxacyclohexyl)-isoxazol | I/ 9 | 66 | 88/0,05 | |
| 18 | 5-Hydroxymethyl-3-(3-oxacyclopentyl)-isoxazol | I/10 | 81 | 107/0,1 | |
| 19 | 5-Hydroxymethyl-3-(2-oxacyclopentyl)-isoxazol | I/11 | 68 | 84-88/0,01 | |
| 20 | 5-Hydroxymethyl-3-cyclopentyl-isoxazol | I/12 | 79 | 78/0,2 | |
| 21 | 4-Hydroxymethyl-3,5-dimethyl-isoxazol | I/13 | 75 | 84-86/4 | |
| 22 | 3-Hydroxymethyl-5-isopropyl-isoxazol | I/14 | 73 | 50/0,2 | ohne KBr |
| 23 | 4-Hydroxymethyl-pyridin | I/15 | 73 | 80/20 | ohne KBr |
| 24 | 3-Hydroxymethyl-pyridin | I/16 | 77 | 96/20 | ohne KBr |

Beispiel 25

Herstellung von 3-(1-Oxohexyl)-pyridin

Eine Lösung aus 3,9 g (21,1 mmol) Triacetonamin und 60 ml Methylenchlorid wurde bei 25 °C mit einer Lösung aus 10,8 g (62,1 mmol) m-Chlorperbenzoesäure und 120 ml Methylenchlorid versetzt und 20 min gerührt. Anschließend wurde eine Lösung aus 120 ml Methylenchlorid, 300 ml Wasser, 3 g (25,2 mmol) Kaliumbromid, 7,8 g (49,8 mmol) $NaH_2PO_4 \cdot 2 H_2O$ und 89,5 g (0,503 mol) 3-(1-Hydroxyhexyl)-pyridin hinzugegeben. Der pH-Wert der wäßrigen Phase wurde mit Salzsäure oder Natronlauge auf pH = 7,5 eingestellt. Unter intensivem Rühren wurden 306 g einer 14 gew.%igen wäßrigen Natriumhypochloridlösung (0,58 mol NaOCl) zugetropft. Nach 30 min Rühren wurde das Reaktionsgemisch mit Salzsäure oder Natronlauge auf pH = 8,5 eingestellt, wonach die wäßrige Phase abgetrennt und zweimal mit je 150 ml Methylenchlorid extrahiert wurde. Die vereinigten organischen Phasen wurden wie üblich aufgearbeitet.
Ausbeute: 73 %
Sdp.: 90 °C/0,3 mbar

## Patentansprüche

1. Verfahren zur Herstellung von heterocyclisch-aromatischen (heteroaromatischen) Aldehyden und Ketonen der allgemeinen Formel I

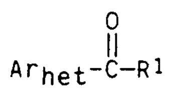

I

in der $Ar_{het}$ einen 5- oder 6-gliedrigen heteroaromatischen Rest mit mindestens einem Stickstoffatom und $R^1$ Wasserstoff oder einen $C_1$-$C_{20}$-Alkylrest bedeutet, durch Oxidation der entsprechenden 1-Hydroxyalkylverbindungen II

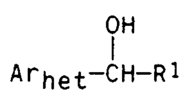

II

dadurch gekennzeichnet, daß man hierzu als Oxidationsmittel ein anorganisches oder organisches Hypochlorit oder Hypobromit (III) in Verbindung mit einem $\alpha,\alpha,\omega,\omega$-Tetraalkylcycloalkan der allgemeinen Formel IV

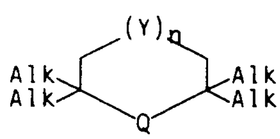

IV

verwendet, in der Alk gleiche oder verschiedene $C_1$-$C_4$-Alkylgruppen bezeichnet, Q für eine der Gruppierungen

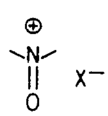

;

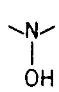

oder

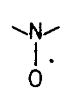

steht, wobei $X^-$ ein Anion bedeutet, n 0 oder 1 ist und Y für Sauerstoff, die Carbonylgruppe oder einen Rest

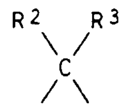

steht, in welchem $R^2$ und $R^3$ Wasserstoff, die Hydroxylgruppe oder C-organische oder O-organische Reste bedeuten, die auch miteinander zu einem fünf- oder sechsgliedrigen Ring verbunden sein können.

**2.** Verfahren nach Anspruch 1, dadurch gekennzeichnet, daß $Ar_{het}$ in den Verbindungen I bzw. II ein unsubstituierter oder substituierter Isoxazolyl-, Pyridin- oder Pyrimidinrest ist.

**3.** Verfahren nach den Ansprüchen 1 und 2, dadurch gekennzeichnet, daß man es in Gegenwart von Natrium- oder Kaliumbromid ausführt.

**4.** Verfahren nach den Ansprüchen 1 bis 3, dadurch gekennzeichnet, daß man die Umsetzung in einem zweiphasigen wäßrig-organischen Flüssigkeitssystem vornimmt.

**5.** Verfahren nach den Ansprüchen 1 bis 4, dadurch gekennzeichnet, daß man die Umsetzung bei pH 5 bis 10 vornimmt.

**6.** Verfahren nach den Ansprüchen 1 bis 5, dadurch gekennzeichnet, daß man die Verbindungen IV in situ aus den entsprechenden Pyrrolidinen bzw. Piperidinen und einem Oxidationsmittel herstellt.

## Claims

**1.** A process for the preparation of a heterocyclic aromatic (heteroaromatic) aldehyde or ketone of the formula I.

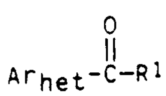

I

where $Ar_{het}$ is a 5- or 6-membered heteroaromatic radical containing at least one nitrogen atom, and $R^1$ is hydrogen or $C_1$-$C_{20}$-alkyl, by oxidizing the corresponding 1-hydroxyalkyl compound II

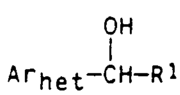

II

which comprises using, as oxidant, an inorganic or organic hypochlorite or hypobromite (III) in combination with an $\alpha,\alpha,\omega,\omega$,-tetralkylcycloalkane of the formula IV

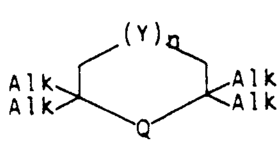

IV

where the Alk radicals are identical or different $C_1$-$C_4$-alkyl groups, Q is one of the groups

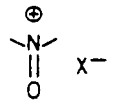 ; 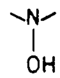 or 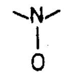

where $X^-$ is an anion, n is 0 or 1, and Y is oxygen, the carbonyl group or

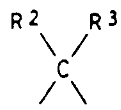

in which $R^2$ and $R^3$ are hydrogen, hydroxyl or C-organic or O-organic radicals and may also be connected to one another to form a five- or six-membered ring.

**2.** A process as claimed in claim 1, wherein $Ar_{het}$ in the compounds I and II is unsubstituted or substituted isoxazolyl, pyridyl or pyrimidyl.

**3.** A process as claimed in claim 1 or 2, wherein the reaction is carried out in the presence of sodium bromide or potassium bromide.

**4.** A process as claimed in claim 1 or 2 or 3, wherein the reaction is carried out in a two-phase aqueous/organic liquid system.

**5.** A process as claimed in claim 1 or 2 or 3 or 4, wherein the reaction is carried out at pH 5 to 10.

**6.** A process as claimed in claim 1 or 2 or 3 or 4 or 5, wherein the compound IV is prepared in situ from the corresponding pyrrolidine or piperidine and an oxidant.

**Revendications**

**1.** Procédé de préparation d'aldéhydes et de cétones hétérocycliques-aromatiques (hétéro-aromatiques) de formule générale I

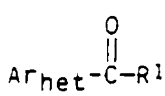 I

dans laquelle $Ar_{hét}$ représente un reste hétéro-aromatique à 5 ou 6 chaînons portant au moins un atome d'azote et $R^1$ hydrogène ou un reste alkyle en C 1-C 20, par oxydation des composés 1-hydroxy-alkyliques II correspondants

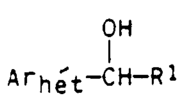 II

caractérisé par le fait que l'on utilise, comme agent d'oxydation, un hypochlorite ou hypobromite (III) inorganique ou organique en combinaison avec un -tétraalkylcycloalcane de formule générale IV

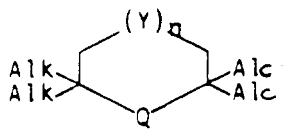 IV

dans laquelle Alc désigne des groupes alkyle en C 1-C 4 identiques ou différents, Q est mis pour un des groupements

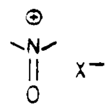 ; 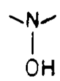 ou 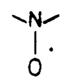

$X^-$ représentant un anion, n étant 0 ou 1 et Y étant mis pour oxygène, le groupe carbonyle ou un reste

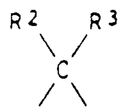

dans lequel $R^2$ et $R^3$ représentent hydrogène, le groupe hydroxyle ou des restes C-organiques ou O-organiques pouvant aussi être reliés entre eux en formant un noyau pentagonal ou hexagonal.

**2.** Procédé selon la revendication 1, caractérisé par le fait que $Ar_{hét}$ dans les composés I ou II, représente un reste isoxazolyle, pyridine ou pyrimidine non substitué ou substitué.

**3.** Procédé selon les revendications 1 et 2, caractérisé par le fait qu'on l'effectue en présence de bromure de sodium ou de bromure de potassium.

**4.** Procédé selon les revendications 1 à 3, caractérisé par le fait que l'on effectue la réaction dans un système de liquide organique aqueux à deux phases.

**5.** Procédé selon les revendications 1 à 4, caractérisé par le fait que l'on effectue la réaction à un pH compris entre 5 et 10.

**6.** Procédé selon les revendications 1 à 5, caractérisé par le fait que l'on prépare les composés IV in situ, à partir des pyrrolidines ou pipéridines correspondantes et un oxydant.